**Europäisches Patentamt**

**European Patent Office**

**Office européen des brevets**

(19)

(11) Veröffentlichungsnummer: **0 030 209**
**B1**

(12) **EUROPÄISCHE PATENTSCHRIFT**

(45) Veröffentlichungstag der Patentschrift:
22.02.84

(51) Int. Cl.³: **C 07 D 249/08**

(21) Anmeldenummer: 80810333.7

(22) Anmeldetag: 03.11.80

(54) Verfahren zur Herstellung von 1H-1,2,4-Triazol.

(30) Priorität: 07.11.79 US 92257

(43) Veröffentlichungstag der Anmeldung:
10.06.81 Patentblatt 81/23

(45) Bekanntmachung des Hinweises auf die Patenterteilung:
22.02.84 Patentblatt 84/8

(84) Benannte Vertragsstaaten:
AT BE CH DE FR GB IT LI LU NL SE

(56) Entgegenhaltungen:
DE - A - 2 802 491

THE JOURNAL OF THE AMERICAN CHEMICAL
SOCIETY, Band LXXVII, Januar bis März 1955, Easton,
C. AINSWORTH et al. "Isomeric and
Nuclear-Substituted beta-Aminoethyl-1,2,4-Triazoles"
Seiten 621 bis 624

(73) Patentinhaber: **CIBA-GEIGY AG, Patentabteilung**
**Postfach, CH-4002 Basel (CH)**

(72) Erfinder: **Petree, Harris E., 1040, Kingsridge Road,**
**Kernersville, N.C. 27284 (US)**
Erfinder: **Pociask, Joseph R., 809 Meade Drive,**
**Greensboro, N.C. 27410 (US)**
Erfinder: **Gupton, John T., 1039 Crystal Bowl Circle,**
**Casselberry, Florida 32707 (US)**

Verfahren zur Herstellung von 1H-1,2,4-Triazol

Die vorliegende Erfindung betrifft ein Verfahren zur Herstellung von 1H-1,2,4-Triazol durch Umsetzung von Hydrazin und Formamid.

1H-1,2,4-Triazol ist ein wertvolles Zwischenprodukt für die Herstellung von Pflanzenschutzmitteln, insbesondere von Fungiziden. Solche Mittel, ihre Herstellung und Verwendung sind in den US-A-3 912 752 und US-A-4 079 062 beschrieben.

Es ist bekannt, 1H-1,2,4-Triazol in der Weise herzustellen, daß man äquivalente Mengen Formylhydrazin und Formamid langsam auf 100°C erwärmt, wobei sich Wasser und Ammoniak entwickeln, und die Temperatur nach $^1/_2$ Stunden auf 260°C steigert, bei welcher Temperatur 1H-1,2,4-Triazol überdestilliert. Nach dieser Methode wird 1H-1,2,4-Triazol in einer Ausbeute von etwa 50% der Theorie erhalten (G. Pellizari, Gazz. chim. ital. 24, 2, 222−229 (1894) und Ber. dtsch. Chem. Ges. 27 (R) 801 (1894)).

Es ist ferner bekannt, Hydrazin-Hydrat (1 Mol) und Formamid (2 Mol) in einer ersten Reaktionsstufe zu Diformylhydrazin umzusetzen und dieses dann in einer zweiten Reaktionsstufe in flüssigem Ammoniak in einem geschlossenen System unter Druck durch 24stündiges Erhitzen auf 200°C in 1H-1,2,4-Triazol überzuführen. Dabei wird 1H-1,2,4-Triazol in einer Ausbeute von 65% der Theorie bezogen auf eingesetztes Hydrazin-Hydrat erhalten (J. Amer. Chem. Soc. 77, 622, (1955)).

Weiterhin ist bekannt, 1H-1,2,4-Triazol durch Erhitzen von 1 Mol Hydrazin-Hydrat mit etwa 3 Mol Formamid auf 190−260°C herzustellen. Hierzu wird das Gemisch aus Hydrazin-Hydrat und Formamid zunächst zur Bildung von Diformylhydrazin auf 70−120°C erhitzt. Anschließend wird das erhaltene Gemisch von Diformylhydrazin und Formamid auf Temperaturen bis 260°C, vorzugsweise 180−220°C erhitzt, wobei 1H-1,2,4-Triazol in einer Ausbeute von 80−90% der Theorie und in einer Reinheit von etwa 90% gebildet wird. Zur Erzielung guter Ausbeuten ist es notwendig, die Reaktion in einer dreistufigen Kaskade durchzuführen (LU-A-61 617).

Ferner ist ein Verfahren zur Herstellung von 1H-1,2,4-Triazol bekannt, bei dem Formamid und Hydrazin-Hydrat bei 100−250°C im Molverhältnis von 1:2 bis 1:2,7 in Gegenwart von Ammoniak umgesetzt werden. Das Verfahren wird in einer dreistufigen Kaskade durchgeführt, wobei in der ersten Stufe bei 100−120°C, in der zweiten Stufe bei 180−200°C und in der dritten Stufe bei 210−230°C gearbeitet wird und die bei 110−140°C kondensierbaren flüchtigen Reaktionsprodukte der zweiten Stufe in die zweite Stufe und die bei 40−80°C kondensierbaren flüssigen Reaktionsprodukte der dritten Stufe in die erste Stufe zurückgeführt werden. Nach diesem Verfahren wird 1H-1,2,4-Triazol in einer Ausbeute von 93−94% der Theorie und in einer Reinheit von 90−98% erhalten (DE-A-2 802 491).

Wie die obige Übersicht über den Stand der Technik zeigt, lassen sich mit den bekannten Verfahren gute Ausbeuten und gute Reinheit nur durch großen apparativen Aufwand und komplizierte Verfahrensmaßnahmen, wie Durchführung des Verfahrens in einer Rührkesselkaskade und Rückführung von in den einzelnen Stufen gebildeten Nebenprodukten, erreichen. Es ist daher das Ziel der vorliegenden Erfindung, ein Verfahren vorzusehen, nach dem 1H-1,2,4-Triazol in einfacher Weise in guter Ausbeute und Reinheit hergestellt werden kann.

Erfindungsgemäß wird 1H-1,2,4-Triazol dadurch hergestellt, daß man Hydrazin in wasserfreier oder wasserhaltiger Form bei erhöhter Temperatur mit Formamid umsetzt, wobei das Molverhältnis von Formamid zu Hydrazin mindestens 2,5 : 1 beträgt. Das Verfahren ist dadurch gekennzeichnet, daß man das Hydrazin mit solcher Geschwindigkeit in das auf 160−180°C vorerhitzte Formamid einträgt, daß die Reaktionstemperatur aufrecht erhalten bleibt, die gebildeten Nebenprodukte Wasser, Ammoniak und Ameisensäure unmittelbar während der Umsetzung durch Destillation abtrennt, das Reaktionsgemisch nach beendigter Zugabe des Hydrazins 1−2 Stunden bei Reaktionstemperatur hält, anschließend das nicht umgesetzte Formamid abdestilliert und das 1H-1,2,4-Triazol als Rückstand gewinnt.

Da wasserfreies Hydrazin schwierig herzustellen und wegen seiner geringeren Stabilität bei den erfindungsgemäß angewendeten Reaktionstemperaturen und seiner toxischen Eigenschaften schwierig zu handhaben ist, ist die Verwendung von wasserhaltigem Hydrazin bevorzugt. Der Wassergehalt des Hydrazins kann 10−50 Gew.-% betragen. Als besonders vorteilhaft hat sich Hydrazin-Hydrat erwiesen, das aufgrund seiner Zusammensetzung als 64%ige Lösung von Hydrazin in Wasser angesehen werden kann.

Wie oben bereits ausgeführt, beträgt das Molverhältnis von Formamid zu Hydrazin mindestens 2,5 : 1. Vorzugsweise beträgt das Molverhältnis von Formamid zu Hydrazin mindestens 4 : 1. Die Größe des molaren Überschusses an Formamid ist für den Ablauf des Verfahrens an sich nicht kritisch. Sie wird in erster Linie durch ökonomische Gesichtspunkte bestimmt, da mit wachsendem Molverhältnis von Formamid zu Hydrazin die Menge des nach beendigter Umsetzung durch Destillation abzutrennenden Formamids und damit der zur Durchführung des Verfahrens erforderliche Energiebedarf zunimmt. In der Praxis hat es sich als vorteilhaft erwiesen, Molverhältnisse von Formamid zu Hydrazin anzuwenden, die zwischen 4 : 1 und 8 : 1 liegen. Besonders vorteilhaft ist die Verwendung von Molverhältnissen von Formamid zu Hydrazin von 4,5 : 1 bis 6 : 1.

Die Art der Zugabe des Hydrazins zu dem auf Reaktionstemperatur erhitzten Formamid ist für die Durchführbarkeit des erfindungsgemäßen Verfahrens besonders wichtig. Das Hydrazin wird erfindungsgemäß dem auf Reaktionstemperatur vorerhitzen Formamid mit einer derartigen Geschwindigkeit zugesetzt, daß die Reaktionstemperatur aufrechterhalten bleibt. Die Zugabe nimmt in der Regel mehrere Stunden in Anspruch. Als besonders vorteilhaft hat sich eine Zugabezeit des Hydrazins von 2−4 Stunden erwiesen. Es ist ferner vorteilhaft, das Hydrazin in dünnem Strahl unter Rühren unterhalb der Oberfläche des auf Reaktionstemperatur erhitzten Formamids einzuleiten. Unter diesen Bedingungen erfolgt die Reaktion zwischen Formamid und Hydrazin sehr rasch. Durch diese rasche Umsetzung und die sofortige Entfernung der Nebenprodukte Wasser, Ammoniak und Ameisensäure aus dem Reaktionsgemisch ist gewährleistet, daß die Konzentration an Hydrazin stets niedrig ist und daß den intermediär gebildeten Produkten Mono- und Diformylhydrazin als Reaktionspartner im wesentlichen überschüssiges Formamid gegenübersteht. Auf diese Weise wird die bei bekannten Verfahren häufig beobachtete Bildung von Nebenprodukten, wie 4-Amino-1,2,4-triazol und 4-Formamidino-1,2,4-triazol, im wesentlichen vermieden. Nach beendigter Zugabe des Hydrazins wird das Reaktionsgemisch noch 1−2 Stunden bei Reaktionstemperatur nachgerührt.

Wie bereits ausgeführt, wird das erfindungsgemäße Verfahren bei Temperaturen von 160−180°C durchgeführt. Das Verfahren kann grundsätzlich auch bei niedrigeren und höheren Temperaturen durchgeführt werden, jedoch muß bei Temperaturen unter 160°C mit wesentlich verlängerten Reaktionszeiten und bei Temperaturen über 180°C mit zunehmender Zersetzung von Formamid gerechtet werden. Als besonders vorteilhaft hat sich eine Reaktionstemperatur von 168−172°C erwiesen.

Für die Isolierung des nach beendigter Umsetzung im Reaktionsgemisch vorliegenden 1H-1,2,4-Triazols bestehen grundsätzlich verschiedene Möglichkeiten. So kann das 1H-1,2,4-Triazol beispielsweise aus dem überschüssigen Formamid nach Abtrennung des Wassers durch Kristallisation gewonnen werden. Dabei kann das nach der Kristallisation am 1H-1,2,4-Triazol anhaftende Formamid durch Waschen mit einem geeigneten organischen Lösungsmittel, wie Methyläthylketon, Tetrahydrofuran oder Äthylacetat, entfernt werden. Auf diese Weise können jedoch nur 40−50% des im Reaktionsgemisch vorliegenden 1H-1,2,4-Triazols abgetrennt werden, so daß die Notwendigkeit besteht, das 1H-1,2,4-Triazol enthaltende Hydrat zu recyclisieren. Vorzugsweise wird das 1H-1,2,4-Triazol in der Weise isoliert, daß man das überflüssige Formamid durch Destillation bei vermindertem Druck, beispielsweise bei 20−100 Torr, abdestilliert. Bei der destillativen Aufarbeitung des Reaktionsgemisches wird zunächst eine Fraktion mit einem Siedebereich von 95−110°C bei 20−30 Torr erhalten, welches nur etwa 30% Formamid enthält. Aus dieser Fraktion kann durch Redestillation etwa 70% des darin enthaltenen Formamids gewonnen werden. Die zweite Fraktion mit einem Siedebereich von 110−135°C bei 20−30 Torr enthält etwa 85% Formamid und ist direkt für die weitere Umsetzung mit Hydrazin geeignet. Dieses 85%ige Formamid enthält neben etwa 1% 1H-1,2,4-Triazol als Verunreinigungen im wesentlichen Wasser, Ameisensäure und Ammoniumformiat. Im Hinblick auf die Tatsache, daß die Hauptmenge des bei der destillativen Aufarbeitung des Reaktionsgemisches zurückgewonnenen Formamids unmittelbar für die weitere Umsetzung mit Hydrazin verwendet werden kann, stellt die Vakuumdestillation die wirtschaftlichste Form der Aufarbeitung des Reaktionsgemisches dar.

Nach Abtrennung des überschüssigen Formamids durch Vakuumdestillation kann das 1H-1,2,4-Triazol entweder durch direktes Ausgießen der Schmelze auf eine gekühlte Oberfläche und Zerkleinerung der erstarrten Schmelze oder durch Zugabe eines geeigneten Lösungsmittels und Kristallisation aus diesem Lösungsmittel gewonnen werden, wobei durch Kristallisation das reinere Produkt erhalten wird.

Die direkte Gewinnung des 1H-1,2,4-Triazols aus der Schmelze stellt eine sehr einfache Operation dar. Es treten weder Ausbeuteverluste auf, noch werden dem 1H-1,2,4-Triazol weitere Materialien zugesetzt. Das direkt aus der Schmelze gewonnene Triazol ist gewöhnlich 94%ig und enthält 0,5−1,2% 4-Formamidino-1,2,4-triazol und 0,3−0,4% 4-Amino-1,2,4-triazol. Andere Verunreinigungen, wie Wasser, Ameisensäure und Formamid sind in einer gesamten Menge von weniger als 0,6% vorhanden. Bei der Gewinnung des 1H-1,2,4-Triazols aus der Schmelze tritt keine merkliche Zersetzung des Produkts ein. Die Farbe des Produkts variiert von Hellbraun bis Dunkelbraun oder Rotbraun. Wenn weniger gefärbtes Material gewünscht wird, kann das Formamid bei niedrigerem Druck und niedrigerer Temperatur abdestilliert werden. Die Verfärbung des Produkts kann auch durch kontinuierliche Destillation vermindert werden, da hierbei weniger Zersetzung eintritt. Das aus der Schmelze gewonnene 94%ige 1H-1,2,4-Triazol ist jedoch unmittelbar für die Synthese von fungizieden und mikrobiziden Wirkstoffen und anderen Schädlingsbekämpfungsmitteln geeignet.

Das 1H-1,2,4-Triazol kann in reinerer Form gewonnen werden, wenn man die nach Abdampfen des Formamids erhaltene Schmelze aus einem geeigneten Lösungsmittel umkristallisiert. Geeignete Lösungsmittel sind beispielsweise Äthylacetat, Tetrahydrofuran, Methyläthylketon und Formamid. Das durch Kristallisation erhaltene 1H-1,2,4-Triazol ist 96−98%ig und enthält weniger als 0,3% 4-Formamidino-1,2,4-triazol und weniger als 0,1% 4-Amino-1,2,4-triazol. Wenn die Umkristallisation der Schmelze mit Äthylacetat durchgeführt wird, wird das 1H-1,2,4-Triazol in einer durchschnittlichen Ausbeute von 87% der Theorie erhalten. Weitere 9% der Theorie an 1H-1,2,4-Triazol können aus der Mutterlauge gewonnen werden. Hierzu wird das Lösungsmittel aus der Mutterlauge abdestilliert und der Rückstand in Tetrahydrofuran oder Methyläthylketon aufgeschlämmt und filtriert. Man gewinnt auf diese Weise etwa 70% des in der Mutterlauge vorhandenen 1H-1,2,4-Triazols. Insgesamt kann das 1H-1,2,4-Triazol durch Kristallisation bei Aufarbeitung der Mutterlauge in einer Ausbeute von 93—95% der Theorie erhalten werden.

Nach einer bevorzugten Ausführungsform des erfindungsgemäßen Verfahrens erhitzt man Formamid auf 168—172°C und leitet bei dieser Temperatur während 2—4 Stunden unter Rühren Hydrazin-Hydrat unterhalb der Oberfläche des Formamids ein, wobei das Molverhältnis von Formamid zu Hydrazin mindestens 4 : 1 beträgt, destilliert die Nebenprodukte Wasser, Ammoniak und Ameisensäure unmittelbar während der Umsetzung ab und entfernt anschließend das überschüssige Formamid durch Vakuumdestillation und gewinnt das 1H-1,2,4-Triazol als Schmelze.

Mit dem erfindungsgemäßen Verfahren wird es möglich, 1H-1,2,4-Triazol auf einfache Weise in sehr guter Ausbeute und Reinheit aus Formamid und Hydrazin herzustellen. Durch die erfindungsgemäße Arbeitsweise wird bei Verwendung eines verhältnismäßig geringen Überschusses an Formamid die Bildung von unerwünschten Nebenprodukten, wie 4-Amino-1,2,4-triazol und 4-Formamidino-1,2,4-triazol weitgehend vermieden. Durch Verwendbarkeit geringer Überschüsse an Formamid wird die erfindungsgemäße Arbeitsweise ermöglicht. Vergleichsweise sei darauf hingewiesen, daß bei der Herstellung von 1H-1,2,4-Triazol durch Vermischen von Formamid und Hydrazin-Hydrat und anschließendes Aufheizen auf Reaktionstemperatur ein Molverhältnis von Formamid zu Hydrazin-Hydrat von mindestens 12 : 1 erforderlich ist, um eine Ausbeute von 90% der Theorie zu erreichen. Das erfindungsgemäße Verfahren läßt sich vorteilhaft kontinuierlich durchführen.

Das erfindungsgemäße Verfahren wird durch die folgenden Beispiele erläutert.

### Beispiel 1

#### a) Umsetzung von Hydrazin-Hydrat mit Formamid

In einem 3 Liter Fünfhalsrundkolben werden 1440 g (32 Mol) Formamid auf 170°C erhitzt und mittels eines Temperaturreglers bei 170—172°C gehalten. In das vorerhitzte Formamid werden unter Rühren 400,5 g (8,0 Mol) Hydrazin-Hydrat unterhalb der Oberfläche mit einer Geschwindigkeit von 2—3 Mol/h eingeleitet. Während der Zugabe des Hydrazins erfolgt eine heftige Entwicklung von Ammoniak, Wasser und Ameisensäure, welche über eine Kolonne aus dem Reaktionsgefäß herausdestilliert werden. Dabei wird die Temperatur am Kolonnenkopf unter 100°C gehalten um Verluste an Formamid auf ein Minimum zu beschränken. Nach beendigter Zugabe des Hydrazin-Hydrats wird die Temperatur des Reaktionsgemisches noch weitere 1 1/2 Stunden bei 170°C gehalten. Dann wird das Reaktionsgemisch auf Raumtemperatur abgekühlt. Es werden 1200—1250 g einer gelblichen Lösung erhalten, welche beim Stehen kristallisiert. Die erhaltene Lösung enthält 42—45% 1H-1,2,4-Triazol, was einer in situ-Ausbeute von 95—98% der Theorie entspricht.

#### b) Isolierung des 1H-1,2,4-Triazols aus dem Reaktionsgemisch

Das in Abschnitt a) beschriebene Reaktionsgefäß wird nach 1 1/2stündigem Nachrühren des Reaktionsgemisches bei 170°C an eine Apparatur zur Vakuumdestillation angeschlossen und das Formamid bei 20 Torr (15 mbar) abdestilliert. Als erste Fraktion werden 150—175 ml Destillat erhalten, das bei 105—110°C übergeht. Diese Fraktion enthält etwa 30% Formamid. Die Destillation wird fortgesetzt und es werden als zweite Fraktion 350—400 ml Destillat mit einem Siedepunkt von 115—120°C erhalten. Nun wird die Heizung unterbrochen, um zu verhindern, daß die Temperatur des Rückstandes über 130°C steigt. Es werden 540—570 g einer rötlich-braunen Schmelze erhalten, die 93—95% 1H-1,2,4-Triazol enthält, was einer Ausbeute von 92—97% der Theorie, bezogen auf Hydrazin-Hydrat entspricht. Die Schmelze wird nach dem Erstarren zerkleinert.

#### c) Umkristallisation des rohen 1H-1,2,4-Triazols

Eine nach der in Abschnitt b) beschriebenen Methode erhaltene Schmelze von rohem 1H-1,2,4-Triazol wird auf etwa 80°C abgekühlt und im Reaktionsgefäß nach Zugabe einer hinreichenden Menge eines Lösungsmittels, wie Methyläthylketon, Tetrahydrofuran, Äthylacetat oder Formamid, unter Erwärmen und Rühren vollständig in Lösung gebracht. Die erhaltene Lösung wird dann auf 10°C abgekühlt, wobei ein Kristallbrei entsteht, aus dem das kristalline 1H-1,2,4-Triazol durch Filtration abgetrennt wird. Nach dem Trocknen erhält man 480—510 g eines bräunlichen bis rötlichen Produkts, das 96—98% 1H-1,2,4-Triazol enthält, was einer Ausbeute von 84—90% der Theorie entspricht. Aus der Mutterlauge kann durch Abdampfen eines Teils des Lösungsmittels eine zweite Fraktion 1H-1,2,4-Triazol gewonnen werden, die abfiltriert und mit Tetrahydrofuran oder Methyläthylketon gewaschen wird. Das auf diese Weise mit einer Gesamtausbeute von 92—94% der Theorie erhaltene 1H-1,2,4-Triazol kann entweder als solches verwendet oder weiter gereinigt werden.

Beispiel 2

a) Nach der in Beispiel 1a beschriebenen Methode werden 2552 g (40 Mol) Formamid und 400,5 g (8 Mol) Hydrazin-Hydrat umgesetzt. Nach beendigter Umsetzung und Abkühlen auf Raumtemperatur werden 2300 g einer gelblichen Lösung erhalten, die 23,5% 1H-1,2,4-Triazol enthält, was einer in situ-Ausbeute von 97,8% der Theorie, bezogen auf Hydrazin entspricht.

b) Aus der nach a) erhaltenen Lösung wird das überschüssige Formamid nach der in Beispiel 1 b) beschriebenen Methode abdestilliert. Es werden 560 g Rohprodukt erhalten, das 96% 1H-1,2,4-Triazol enthält, was einer Ausbeute von 97,3%, bezogen auf Hydrazin entspricht.

c) Das nach b) erhaltene Rohprodukt wird aus Äthylacetat umkristallisiert. Man erhält 496 g gelbliches Produkt, das 98% 1H-1,2,4-Triazol enthält, was einer Ausbeute von 87,9% der Theorie, bezogen auf Hydrazin entspricht. Aus der eingeengten Mutterlauge werden weitere 30,5 g bräunliches Produkt erhalten, das 95% 1H-1,2,4-Triazol enthält. Gesamtausbeute 95% der Theorie, bezogen auf Hydrazin.

## Patentansprüche

1. Verfahren zur Herstellung von 1H-1,2,4-Triazol durch Umsetzung von Hydrazin in wasserfreier oder wasserhaltiger Form mit Formamid bei erhöhter Temperatur, wobei das Molverhältnis von Formamid zu Hydrazin mindestens 2,5 : 1 beträgt, dadurch gekennzeichnet, daß man das Hydrazin mit solcher Geschwindigkeit in das auf 160 – 180°C vorerhitzte Formamid einträgt, daß die Reaktionstemperatur aufrechterhalten bleibt, die gebildeten Nebenprodukte Wasser, Ammoniak und Ameisensäure unmittelbar während der Umsetzung durch Destillation abtrennt, das Reaktionsgemisch nach beendigter Zugabe des Hydrazins 1 – 2 Stunden bei Reaktionstemperatur hält, anschließend das nicht umgesetzte Formamid abdestilliert und das 1H-1,2,4-Triazol als Rückstand gewinnt.

2. Verfahren nach Anspruch 1, dadurch gekennzeichnet, daß man das Hydrazin in Form seines Hydrats verwendet.

3. Verfahren nach Anspruch 1, dadurch gekennzeichnet, daß man Formamid und Hydrazin in einem Molverhältnis zwischen 4 : 1 und 8 : 1 verwendet.

4. Verfahren nach Ansprüchen 1 und 3, dadurch gekennzeichnet, daß man Formamid und Hydrazin in einem Molverhältnis von 4,5 : 1 bis 6 : 1 verwendet.

5. Verfahren nach Anspruch 1, dadurch gekennzeichnet, daß man das Hydrazin während 2 – 4 Stunden unterhalb der Oberfläche des auf Reaktionstemperatur erhitzten Formamids einleitet.

6. Verfahren nach Anspruch 1, dadurch gekennzeichnet, daß man die Umsetzung von Formamid und Hydrazin bei einer Temperatur von 168 – 172°C durchführt.

7. Verfahren nach Anspruch 1, dadurch gekennzeichnet, daß man nach beendigter Umsetzung das überschüssige Formamid bei vermindertem Druck abdestilliert.

8. Verfahren nach Anspruch 1, dadurch gekennzeichnet, daß man das nach der destillativen Abtrennung des überschüssigen Formamids erhaltene Rohprodukt aus Äthylacetat, Tetrahydrofuran, Methyläthylketon oder Formamid umkristallisiert.

9. Verfahren nach Anspruch 1, dadurch gekennzeichnet, daß man das Formamid auf 168 – 172°C erhitzt und bei dieser Temperatur während 2 – 4 Stunden unter Rühren Hydrazin-Hydrat unterhalb der Oberfläche des Formamids einleitet, wobei das Molverhältnis von Formamid zu Hydrazin-Hydrat mindestens 4 : 1 beträgt, und das überschüssige Formamid durch Vakuumdestillation abtrennt.

## Claims

1. A process for producing 1H-1,2,4-triazole by reaction of hydrazine, in the anhydrous or hydrous form, with formamide at elevated temperature, the molar ratio of formamide to hydrazine being at least 2.5 : 1, which process comprises introducing the hydrazine at such a rate into the formamide preheated to 160 – 180°C, that the reaction temperature is maintained, separating the formed by-products, water, ammonia and formic acid, directly by distillation during the reaction, holding the reaction mixture, after completion of the addition of hydrazine, for 1 – 2 hours at the reaction temperature, distilling off the unreacted formamide, and obtaining the formed 1H-1,2,4-triazole as residue.

2. A process according to Claim 1, wherein the hydrazine is used in the form of its hydrate.

3. A process according to Claim 1, wherein formamide and hydrazine are used in a molar ratio of between 4 : 1 and 8 : 1.

4. A process according to Claims 1 and 3, wherein formamide and hydrazine are used in a molar ratio of 4.5 : 1 to 6 : 1.

5. A process according to Claim 1, wherein the hydrazine is introduced, in the course of 2 – 4 hours, below the surface of the formamide heated to reaction temperature.

6. A process according to Claim 1, wherein the reaction of formamide and hydrazine is performed at a temperature of 168 – 172°C.

7. A process according to Claim 1, wherein the unreacted formamide, after completion of the reaction, is distilled off under reduced pressure.

8. A process according to Claim 1, wherein the crude product obtained after removal of the unreacted formamide by distillation is recrystallised from ethyl acetate, tetrahydrofuran, methyl ethyl ketone or formamide.

9. A process according to Claim 1, wherein the formamide is heated to 168—172°C; hydrazine hydrate is introduced at this temperature for 2—4 hours, with stirring, below the surface of the formamide, the molar ratio of formamide to hydrazine hydrate being at least 4 : 1; and subsequently removing the unreacted formamide by vacuum distillation.

## Revendications

1. Procédé de préparation de 1H-1,2,4-triazole par réaction d'hydrazine sous forme anhydre ou aqueuse avec du formamide à température accrue, le rapport molaire du formamide à l'hydrazine s'élevant à au moins 2,5 : 1, caractérisé en ce qu'on introduit l'hydrazine dans le formamide préalablement chauffé à 160—180°C avec une vitesse telle que la température de la réaction reste constante, en ce qu'on sépare par distillation les produits secondaires eau, ammoniac et acide formique formés immédiatement pendant la réaction, en ce qu'on maintient le mélange réactionnel à la température de la réaction pendant 1 à 2 h après la fin de l'addition de l'hydrazine, puis en ce qu'on sépare par distillation le formamide n'ayant pas réagi et en ce qu'on obtient comme résidu le 1H-1,2,4-triazole.

2. Procédé selon la revendication 1, caractérisé en ce qu'on utilise l'hydrazine sous la forme de son hydrate.

3. Procédé selon la revendication 1, caractérisé en ce qu'on utilise le formamide et l'hydrazine dans un rapport molaire compris entre 4 : 1 et 8 : 1.

4. Procédé selon les revendications 1 et 3, caractérisé en ce qu'on utilise le formamide et l'hydrazine dans un rapport molaire compris entre 4,5 : 1 et 6 : 1.

5. Procédé selon la revendication 1, caractérisé en ce qu'on introduit l'hydrazine pendant 2 à 4 h en-dessous de la surface du formamide chauffé à la température de la réaction.

6. Procédé selon la revendication 1, caractérisé en ce qu'on conduit la réaction du formamide et de l'hydrazine à une température de 168—172°C.

7. Procédé selon la revendication 1, caractérisé en ce qu'après la fin de la réaction on distille l'excès de formamide à une pression réduite.

8. Procédé selon la revendication 1, caractérisé en ce qu'après la séparation de l'excès de formamide par distillation on recristallise le produit brut obtenu à partir de l'acétate d'éthyle, du tétrahydrofuranne, de la méthyléthylcétone ou du formamide.

9. Procédé selon la revendication 1, caractérisé en ce qu'on chauffe le formamide à 168—172°C et en ce qu'à cette température on introduit pendant 2 à 4 h tout en agitant de l'hydrazine hydratée en-dessous de la surface du formamide, le rapport molaire du formamide à l'hydrazine hydratée s'élevant à au moins 4 : 1, et en ce qu'on sépare l'excès de formamide par distillation sous vide.